# EUROPÄISCHE PATENTSCHRIFT

(11) **EP 0 761 245 B1**
(45) Veröffentlichungstag und Bekanntmachung des Hinweises auf die Patenterteilung: **11.04.2001**
(21) Anmeldenummer: 96114499.5
(22) Anmeldetag: 10.09.1996
(51) Int. Cl.: A61M 1/10, F04B 43/08

(54) **Extrakorporale Blutpumpe**
Extra-corporeal blood pump
Pompe sanguine extracorporelle

(30) Priorität: 11.09.1995 DE 19533595
(43) Veröffentlichungstag der Anmeldung: 12.03.1997
(73) Patentinhaber: Deco Delta GmbH, 70372 Stuttgart (DE)
(72) Erfinder: Achterholt, Rainer, D-87471 Durach/Weidach (DE)
(74) Vertreter: Brehm, Hans-Peter, Dr. Dipl.-Chem.

(56) Entgegenhaltungen:
- CH-A- 657 670
- FR-A- 1 504 494
- FR-A- 2 299 532

## Beschreibung

Die vorliegende Erfindung betrifft eine extrakorporale Blutpumpe nach dem Oberbegriff des Anspruches 1. Insbesondere betrifft die Erfindung eine solche Blutpumpe, die zur Förderung von Blut durch einen Dialyseapparat vorgesehen ist. Typischerweise beträgt der Durchsatz durch einen Dialyseapparat etwa 100 ml/min. In Einzelfällen kann auch ein Durchsatz bis zu etwa 250 ml/min vorgesehen werden.

Eine extrakorporale Blutpumpe dieser Art ist aus dem Dokument EP-A1-0 418 208 bekannt. Bei der bekannten Blutpumpe ist sowohl eine verschiebliche Anordnung des einlaßseitigen Kopfes wie des auslaßseitigen Kopfes der Pumpenkammer vorgesehen, um diese Köpfe aufeinander zu und voneinander weg zu verschieben. Die eigentliche Pumpenkammer besteht aus einem im wesentlichen kugelförmigen Sack oder Balg, der aus einem flexiblen, jedoch nicht nennenswert dehnbarem Material bestehen soll. Dieser bekannte Sack oder Balg ist so ausgebildet, daß beim aufeinander Zufahren der beiden Köpfe Falten gebildet werden, die teleskopartig aufeinander abgelegt werden. Dank der verschieblichen Anordnung der beiden Köpfe soll beim Füllvorgang der Pumpenkammer ein erheblicher Unterdruck sowie beim Ausstoßvorgang ein erheblicher Überdruck vermieden werden, welcher das Blut schädigen könnte.

Weiterhin offenbart das Dokument EP-A1-0 659 444 eine Vorrichtung zum Pumpen von Blut mit einer pulsierenden Druckblase, welche durch Druckänderungen komprimiert oder expandiert wird und auf diese Weise pulsierend Blut fördert. Diese Druckblase befindet sich innerhalb eines starren, druckdichten Gehäuses, in das mit Hilfe einer Pumpe Druckmedium einbringbar ist, um die Druckblase zu komprimieren oder expandieren zu lassen. Diese bekannte Blutpumpe weist keinen beweglich angeordneten, auslaßseitigen Kopf auf. Weil die Druckblase in einem druckdicht verschließbaren Gehäuse untergebracht ist, dürfte ein Austausch der Druckblase vergleichsweise aufwendig sein.

Das Dokument EP-A1-0 478 499 offenbart eine intermittierend fördernde Pumpe zum Fördern von Blut, bei welcher ein verstellbarer Stoßkörper auf einen druckfesten, deformierbaren Sack einwirkt. Zur Verstellung des Stoßkörpers dient ein elektrischer Hohlwellenmotor, der über eine motorisch angetriebene Spindel und eine Mutter einen Linearantrieb auf den Stoßkörper ausübt.

Die klassische und in weitem Umfang eingesetzte Pumpe zum Fördern von Blut durch einen Dialyseapparat ist die Rollerpumpe, die mit zwei oder drei Rollen ausgerüstet ist, die längs einer Kreisbahn bewegt werden, in welche ein quetschbarer Schlauch eingelegt ist. Jede Rolle quetscht den Druckschlauch so weit, daß dessen Innenwandabschnitte aufeinander zu liegen kommen und der Schlauchquerschnitt geschlossen wird. Mit der fortlaufenden Bewegung der Rollen längs der Kreisbahn wird die vor jeder Quetschstelle befindliche Blutsäule durch das Schlauch-Innenvolumen gefördert. Eine solche "peristaltische" Rollerpumpe zeichnet sich durch eine Reihe von Vorteilen aus. Der mit einem Einlaßanschluß und einem Auslaßanschluß versehene quetschbare Schlauch bildet ein einfaches und preiswert herstellbares Bauteil, das leicht in den Pumpenkörper eingesetzt werden kann und nach Gebrauch gegen einen frischen quetschbaren Schlauch ausgetauscht werden kann. Abgesehen von den Quetschstellen treten keine substantiellen Änderungen im Querschnitt der Blutsäule innerhalb des Schlauches auf. Damit werden erhebliche Scherspannungen, Verwirbelungen und Tot-Wasserzonen innerhalb der gepumpten Blutsäule vermieden. Andererseits ist an einer solchen Rollerpumpe nachteilig, daß im Bereich der Quetschstellen Deformationen und Schädigungen der lebenden Zellen des Blutes, insbesondere der Erythrozyten, Echinozyten, Sphärozyten und Stomatozyten auftreten können. Derartige Deformationen und Schädigungen dieser Zellen können ihrerseits eine Hämolyse zur Folge haben.

Die Aufgabe der vorliegenden Erfindung besteht darin, eine Blutpumpe der eingangs genannten Art zu schaffen, welche weiterhin die vorteilhaften Eigenschaften einer Rollerpumpe beibehält, welche jedoch deren nachteilige Eigenschaften vermeidet, die insbesondere durch den Quetschvorgang des Schlauches hervorgerufen werden.

Die erfindungsgemäße Lösung obiger Aufgabe ist durch eine extrakorporale Blutpumpe gemäß Anspruch 1 gegeben.

Bei einer bevorzugten Ausführungsform der erfindungsgemäßen Blutpumpe ist vorgesehen, daß
- die Einlaßbohrung einen Innendurchmesser aufweist; die Auslaßbohrung einen Innendurchmesser aufweist; und
- das die Pumpenkammer begrenzende Schlauchstück einen Innendurchmesser aufweist, der nicht größer ist als der fünffache Innendurchmesser der Einlaßborhung und/ oder der Auslaßbohrung.

Bei einer weiteren bevorzugten Ausführungsform ist vorgesehen, daß die ursprüngliche Länge des nicht gedehnten Schlauchstückes größer ist als der Innendurchmesser des nicht gedehnten Schlauchstückes.

Bei einer weiteren bevorzugten Ausführungsform ist vorgesehen, daß der Antrieb eine aufeinanderfolgende Hin- und Herbewung des auslaßseitigen Kopfes im wesentlichen in Richtung der Schlauchstück-Längsachse ausführt, wobei pro Hub die ursprüngliche Länge des Schlauchstückes um wenigstens 100 % und um nicht mehr als etwa 400 % der ursprünglichen Schlauchstücklänge gedehnt wird.

Mit dieser Ausgestaltung der Erfindung wird eine einfach aufgebaute Blutpumpe bereitgestellt, deren Pumpenkammer nicht länger gequetscht werden muß, um das Blut zu fördern. Zwischen einerseits dem Innendurchmesser des dehnbaren Schlauches und andererseits der Einlaßbohrung und/oder der Auslaßbohrung treten keine substantiellen Querschnittsänderungen auf, die Anlaß zu Scherspannungen, Verwirbelungen und Tot-Wasserzonen des Blutes bilden könnten. Die einzelnen Übergänge sind glatt, sanft und stetig ausgebildet. Das Bauteil aus dem dehnbaren Schlauchstück mit den beiden endständigen Köpfen und deren Ventileinrichtungen bildet ein selbständig handhabbares, einfach aufgebautes und daher preiswert fertigbares Bauteil, das leicht in das Gestell der Blutpumpe eingesetzt und bei Bedarf gegen ein neues Bauteil dieser Art ausgetauscht werden kann. Insoweit stellt dieses Bauteil einen preiswerten, für den einmaligen Gebrauch bestimmten Wegwerfartikel dar, der nach Gebrauch verworfen wird. Damit entfällt eine Sterilisation der Pumpenkammer. Mit der Erfindung werden die Nachteile der bekannten Rollerpumpe beseitigt, jedoch deren Vorzüge beibehalten. Damit löst die Erfindung die zugrunde liegende Aufgabe.

Vorteilhafte Ausgestaltungen und Weiterbildungen der Erfindung ergeben sich aus den Unteransprüchen.

Bei der erfindungsgemäßen Blutpumpe wird die Pumpwirkung dadurch erzeugt, daß der auslaßseitige Kopf aufeinanderfolgend eine Vorwärtsbewegung unter Streckung des Schlauchstückes und eine Rückwärtsbewegung unter Verkürzung des Schlauchstückes ausführt. Diese periodische Vorwärtsbewegung wird durch den Antrieb bewirkt. Bei einer bevorzugten Ausführungsform wird die periodische Rückwärtsbewegung durch eine inhärente elastische Rückstellkraft des gedehnten Schlauchstückes bewirkt. Bei einer bevorzugten alternativen Ausführungsform kann auch diese Rückwärtsbewegung unter der Wirkung des motorischen Antriebs erzeugt und/oder kontrolliert werden.

Eine periodisch Vorwärtsbewegung des auslaßseitigen Kopfes erzeugt innerhalb der Pumpenkammer der erfindungsgemäßen Blutpumpe eine Saugwirkung. Vorzugsweise ist die Einlaß-Ventileinrichtung als ein Rückschlagventil ausgebildet, das unter dieser Saugwirkung eine Blutströmung aus einer an den Einlaßanschluß angeschlossenen Versorgungsleitung durch die Einlaßbohrung in die Pumpenkammer erzeugt. Weiterhin ist die Auslaß-Ventileinrichtung vorzugsweise als Rückschlagventil ausgebildet, das unter dieser Saugwirkung die Auslaßbohrung verschließt. In gleicher Weise erzeugt eine periodische Rückwärtsbewegung des auslaßseitigen Kopfes innerhalb der Pumpenkammer eine Pumpwirkung. Die als Rückschlagventil ausgebildete Einlaß-Ventileinrichtung verschließt unter dieser Pumpwirkung die Einlaßbohrung. Die als Rückschlagventil ausgebildete Auslaß-Ventileinrichtung erzeugt unter dieser Pumpwirkung eine Blutströmung aus der Pumpenkammer durch die Auslaßbohrung in eine Abgabeleitung, die an den Auslaßanschluß angeschlossen ist.

Nach einer weiteren bevorzugten Ausführungsform besteht das die Pumpenkammer bildende dehnbare Schlauchstück aus einem Gummi-elastischen Material, das für medizinische Zwecke geeignet ist. Zu geeigneten Materialien gehören Silikonmaterialien, Pulyurethanmaterialien und Latexmaterialien. Insbesondere eine Latexfolie mit einer Schichtdicke von etwa 0,4 mm kann wenigstens bis zu 600 % ihrer ursprünglichen Länge gedehnt werden und weist trotzdem eine ausreichende Dauerhaftigkeit und Belastbarkeit auf, um wenigstens einigen tausend Hubvorgängen in einer erfindungsgemäßen Blutpumpe standzuhalten. Gummi-elastische Materialien dieser Art sind im medizinischen Bereich bekannt, werden dort beispielsweise zur Herstellung medizinischer Handschuhe eingesetzt, und stehen handelsüblich zur Verfügung.

Vorzugsweise kann die Blutpumpe ein längliches Gestell aufweisen, das an einem Ende mit einer fest angebrachten Aufnahme für den einlaßseitigen Kopf versehen ist, und das weiterhin eine verschieblich angeordnete Aufnahme aufweist, die in einer Führung oder Nut geführt ist. Diese bewegliche Aufnahme ist zur Halterung des auslaßseitigen Kopfes bestimmt. Zweckmäßigerweise weisen die beiden Aufnahmen eine unterschiedliche Innenkontur, beispielsweise einmal eine runde und zum anderen eine eckige Innenkontur auf, und die zugeordneten Köpfe weisen eine angepaßte Außenkontur auf, so daß nur der passende Kopf in die passende Aufnahme eingesetzt werden kann. Diese Ausgestaltung erlaubt auf einfache Weise ein richtiges Einsetzen der Pumpenkammer in die restliche Blutpumpe und gewährleistet eine zuverlässige, geführte Bewegung des auslaßseitigen Kopfes.

In diesem Falle ist als Antrieb vorzugsweise ein elektrischer Schrittmotor vorgesehen, der eine Spindel antreibt, die in eine Mutter eingreift, welche mit der Aufnahme für den auslaßseitigen Kopf gekoppelt ist, so daß bei motorisch angetriebener Spindel der auslaßseitige Kopf von dem stationären Kopf weg bewegt wird und das Schlauchstück gedehnt wird. Schrittmotoren dieser Art stehen handelsüblich zur Verfügung. Ein Schrittmotor ermöglicht eine leicht kontrollierbare Rotation der Spindel und damit eine zuverlässige Verstellung des auslaßseitigen Kopfes.

Vorzugsweise kann die Rückführbewegung des auslaßseitigen Kopfes durch die elastische Rückstellkraft des gedehnten Schlauchmaterials bewirkt werden. In diesem Falle kann der Schrittmotor mit einer Strombremse versehen sein, welche bei dieser Rückführbewegung die Umdrehungsgeschwindigkeit der Spindel kontrolliert. Auf diese Weise kann auch bei der Rückführbewegung eine kontrollierte und geregelte Geschwindigkeit des auslaßseitigen Kopfes gewährleistet werden, ohne daß ein umsteuerbarer Motor benötigt wird.

Ein alternativer Antrieb zur Verstellung des auslaßseitigen Kopfes weist eine motorisch angetriebene rotierbare Scheibe (oder einfach eine motorisch angetriebene Kurbel) auf, von deren Umfangsabschnitt ein Umfangsglied vertikal absteht, das längs einer Kreisbahn umläuft. Der auslaßseitige Kopf ist mit diesem Umfangsglied gekoppelt, so daß ein Umlaufen dieses Umfangsgliedes eine aufeinanderfolgende Streckung und Verkürzung des Schlauchstückes verursacht, das die Pumpenkammer bildet. Beispielsweise kann das Umfangsglied ein Stift sein, der durch eine passende Durchgangsbohrung geführt ist, die im auslaßseitigen Kopf ausgespart ist. In diesem Falle kann der auslaßseitige Kopf einfach auf diesen Stift aufgesteckt werden.

Ein weiterer alternativer Antrieb zur Verstellung des auslaßseitigen Kopfes weist ein motorisch angetriebens Zahnrad auf, das gleichzeitig ein erstes Zahnrad und ein zweites Zahnrad antreibt, die im Abstand zueinander angeordnet sind, beide mit dem motorisch angetriebenen Zahnrad kämmen und je ein abstehendes Umfangsglied aufweisen. In diesem Falle weist die Blutpumpe eine erste Pumpeinrichtung mit erstem einlaßseitigem Kopf, erster Pumpenkammer und erstem auslaßseitigem Kopf auf, sowie eine zweite Pumpeinrichtung mit zweitem einlaßseitigem Kopf, zweiter Pumpenkammer und zweitem auslaßseitigem Kopf auf. Der erste auslaßseitige Kopf ist mit dem Umfangsglied am ersten Zahnrad gekoppelt, und der zweite auslaßseitige Kopf ist mit dem Umfangsglied am zweiten Zahnrad gekoppelt, so daß gleichzeitig eine erste Pumpeinrichtung und eine zweite Pumpeinrichtung betätigt werden.

Insbesondere in Verbindung mit einem Antrieb, der wenigstens ein umlaufendes Umfangsglied aufweist, das zwangsweise eine aufeinanderfolgende Streckung und Verkürzung des Schlauchstückes durchführt, kann es zweckmäßig sein, eine Art Käfig vorzusehen, welcher das Schlauchstück an einer übermäßigen Ausweitung oder Ausbeulung im Verlauf des Verkürzungsschrittes hindert, der zumindest einen leichten Überdruck innerhalb der Pumpenkammer erzeugt, um das Blut in die Abgabeleitung zu drücken. Dieser Käfig kann ein starres zylindrisches Gehäuse oder ein solcher Mantel sein, welcher einstückig mit dem einlaßseitigen Kopf ausgebildet ist. Dieses Gehäuse oder Mantel weist einen Innendurchmesser auf, der geringfügig größer ist, als der Außendurchmesser des nicht gedehnten Schlauchstückes.

Nachstehend wird die Erfindung mehr im einzelnen anhand bevorzugter Ausführungsformen mit Bezugnahme auf die Zeichnungen erläutert; die letzteren zeigen:
- Figur 1: in einer schematischen Seitenansicht eine Pumpeinrichtung mit Pumpenkammer und mit den beiden Köpfen;
- Figur 2: in einer schematischen Seitenansicht eine erfindungsgemäße Blutpumpe, wobei der bewegliche Kopf eine Position einnimmt,welche dem Zustand gerade vor einer Streckung des Schlauchstückes entspricht, um die Pumpenkammer mit Blut zu füllen;
- Figur 3: in einer schematischen Seitenansicht die Blutpumpe nach Fig. 2, wobei der bewegliche Kopf eine Position einnimmt, welche dem Zustand der Pumpenkammer nach dem Streckvorgang und gerade vor der Verkürzung des Schlauchstückes entspricht, um Blut in die Abgabeleitung zu drücken;
- Figur 4a: eine schematische Seitenansicht einer Blutpumpe mit einem Antrieb, der ein umlaufendes Umfangsglied aufweist, das vertikal von einem Umfangsabschnitt einer rotierbaren Scheibe absteht und aufeinanderfolgend das Schlauchstück streckt und verkürzt;
- Figur 4b: in einer ähnlichen Darstellung wie Fig. 4a das Schlauchstück in gestrecktem Zustand;
- Figur 5a: eine schematisch Draufsicht auf eine Blutpumpe mit einem anderen Antrieb, wobei je ein umlaufendes Umfangsglied vertikal von einem Umfangsabschnitt eines ersten angetriebenen Zahnrades und eines zweiten angetriebenen Zahnrades absteht, und je das Schlauchstück einer ersten Pumpeinrichtung und einer zweiten Pumpeinrichtung aufeinanderfolgend streckt und verkürzt;
- Figur 5b: in einer ähnlichen Darstellung wie Fig. 5a die beiden Schlauchstücke in gestrecktem Zustand; und
- Figur 6: in einer schematischen Seitenansicht einen Ausschnitt einer Pumpeinrichtung mit einem einlaßseitigen Kopf, der mit einem einstückig angeformten Gehäuse oder Mantel versehen ist, um eine übermäßige Ausweitung des Schlauchstückes zu verhindern.

Die erfindungsgemäße Blutpumpe weist drei Hauptbestandteile auf, nämlich ein Gestell 10, eine als selbständiges Bauteil handhabbare Pumpeinrichtung 30, und einen Antrieb.

Die Pumpeinrichtung 30 besteht aus einem Stück eines dehnbaren Schlauches 32, dem einlaßseitigen Kopf 33 und dem auslaßseitigen Kopf 43. Das dehnbare Schlauchstück 32 besteht aus einem für medizinische Zwecke geeigenten, Gummi-elastischem Material, das wenigstens bis zum Fünffachen seiner ursprünglichen Länge dehnbar bzw. ausziehbar ist, ohne daß eine nennenswerte Einschnürung des Innenvolumens der Pumpenkammer 31 erfolgt. Ein geeignetes und bevorzugtes Material ist ein natürliches Latexmaterial mit einer Schichtdicke von etwa 0,4 mm. Im Regelfalle kann bereits durch geeignete Auswahl der Wandstärke des Schlauchstückes und/oder der Shore-Härte des Schlauchmaterials eine substantielle Einschnürung des Pumpenkammer-Innenvolumens beim Ausziehen bzw. Dehnen des Schlauchstückes verhindert werden. Bei Bedarf kann zusätzlich eine solche Einschnürung verhindert werden durch eine angepaßte, sich über die Länge des Schlauchstückes ändernde Stärke bzw. Dicke der Schlauchwand. Beispielsweise kann in diesem Falle ein Schlauchstück gewählt werden, dessen Wandstärke von der Schlauchmitte in Richtung auf die beiden Schlauchstückenden hin zunimmt. Das Schlauchmaterial soll gegenüber Blut inert sein und darf keine Schlauchbestandteile wie etwa Weichmacher an das Blut abgeben. Vorzugsweise ist die Schlauchoberfläche glatt und weist biokompatible Eigenschaften auf. Als geeignete Materialien kommen beispielsweise Silikonkautuschuk, ausgewählte Polyurethane und natürliche Latexmaterialien in Betracht.

Das Schlauchstück kann einen Innendurchmesser von etwa 4 bis etwa 25 mm aufweisen. Beim Pumpen zum Fördern von Blut durch Oxigenatoren können vorzugsweise Schlauchdurchmesser bis etwa 12 mm ("Halb-Zoll-Schläuche") vorgesehen werden. Noch mehr bevorzugt ist ein Schlauchdurchmesser von etwa 4 mm bis etwa 8 mm, um die Unterschiede zwischen dem Schlauchdurchmesser und den Bohrungen des einlaßseitigen und/oder auslaßseitigen Kopfes möglichst gering zu halten.

Die nicht gestreckte Pumpenkammer 31 soll eine Länge von mehreren Zentimetern aufweisen, um jeglichen Kontakt zwischen der inneren Stirnfläche des einlaßseitigen Kopfes mit derjenigen des auslaßseitigen Kopfes zu vermeiden; auch soll selbst im nicht gedehnten Zustand eine gewisse Menge Blut innerhalb der Pumpenkammer 31 vorhanden sein; dies ist hilfreich, um die Gefahr einer Verwirbelung und/oder turbulenten Blutströmung innerhalb der Pumpenkammer 31 zu minimieren. Um das pro Hub geförderte Blutvolumen zu steigern, ist eher bevorzugt, die Länge der Pumpenkammer 31 zu erhöhen, als den Innendurchmesser der Pumpenkammer 31 zu erhöhen.

Das Schlauchstück 32 weist gegenüberliegende Schlauchstückenden 32', 32" auf. Das eine Schlauchstückende 32 ist dicht und fest mit dem einlaßseitigen Kopf 33 verbunden, und das andere gegenüberliegende Schlauchstückende 32' ist dicht und fest mit dem auslaßseitigen Kopf 43 verbunden. Jeder Kopf 33, 43 bildet einen Spritzgußkörper aus einem biokompatiblen Kunststoff wie etwa Polycarbonat, Polyethylen, Polypropylen oder Polyurethan. Bei Bedarf können die vom Blut kontaktierten Flächen zusätzlich mit einer Beschichtung aus biokompatiblem Kohlenstoffmaterial versehen sein. Am einlaßseitigen Kopf 33 ist einstückig ein Einlaßanschluß 34 angeformt, der eine durchgehende Einlaßbohrung 35 begrenzt, durch welche Blut in den Innenraum der Pumpenkammer 30 strömen kann. Typischerweise dient dieser Einlaßanschluß 34 zum Anschluß eines - nicht dargestellten - Zuführschlauches, der zu einer Nadel führt, welche in die Arterie eines Patienten einführbar ist, oder dieser Zuführschlauch ist mit einem Gefäß zur Zwischenlagerung des Blutes verbunden, das dem Patienten entnommen worden ist. Der Durchmesser der Einlaßbohrung 35 im Einlaßanschluß 34 korrespondiert mit dem Durchmesser dieses Zuführschlauches. Vorzugsweise weist diese Einlaßbohrung 35 einen vergleichsweise großen Innendurchmesser auf, um den Unterschied zum Innendurchmesser der Pumpenkammer 31 möglichst gering zu halten. Innerhalb des einlaßseitigen Kopfes 33 kann eine Erweiterung 36 dieser Einlaßbohrung 35 in Richtung auf die Pumpenkammer 31 zu vorgesehen sein.

Angrenzend an diese Einlaßbohrung 35 oder Erweiterung 36 ist eine Einlaß-Ventileinrichtung 37 ausgebildet. Aufgabe dieser Einlaß-Ventileinrichtung 37 ist es, im Falle eines Unterdruckes innerhalb der Pumpenkammer 31 eine Blutströmung durch die Einlaßbohrung 35 zu ermöglichen und im Falle eines Überdruckes innerhalb der Pumpenkammer 31 eine Blutströmung durch diese Einlaßbohrung 35 zu sperren. Diese Einlaß-Ventileinrichtung 37 kann ein vergleichsweise einfach aufgebautes Rückschlagventil sein, das ein bewegliches Ventilglied aufweist, das beispielsweise aus einer beweglich angebrachten Klappe, Fahne, Segel oder Membran 37 besteht, die mit ihrem Umfangsrandabschnitt auf einer als Ventilsitzfläche dienenden Auflagefläche 38 am einlaßseitigen Kopf 33 aufliegen kann.

Der auslaßseitige Kopf 43 ist im wesentlichen komplementär zu dem oben beschriebenen einlaßseitigen Kopf 33 ausgebildet und weist ebenfalls einen einstückig angeformten Auslaßanschluß 44 auf, der eine Auslaßbohrung 45 begrenzt, die über eine wahlweise vorgesehene Erweiterung 46 mit dem Innenraum der Pumpenkammer 31 in Verbindung steht. An den Auslaßanschluß 44 kann typischerweise ein biegsamer Abgabeschlauch 50 angeschlossen werden, der zu einem - nicht dargestellten - Dialyseapparat oder einer sonstigen Einrichtung des extrakorporalen Blutkreislaufes führt.

Diese Auslaßbohrung 45 kann den gleichen Innendurchmesser aufweisen, wie die Einlaßbohrung 35. Vorzugsweise kann dieser Innendurchmesser der Einlaßbohrung 35 und/oder der Auslaßbohrung 45 etwa 1 bis etwa 6 mm, insbesondere etwa 2 bis etwa 5 mm und besonders bevorzugt etwa 2 mm bis etw 4 mm betragen. Entsprechend einem bevorzugten und wichtigen Merkmal der vorliegenden Erfindung soll der Innendurchmesser des Schlauchstückes 32 nicht mehr als das Fünffache dieses Innendurchmessers der Einlaßbohrung 35 und/oder der Auslaßbohrung 45 betragen. Dieses Merkmal gewährleistet eine sanfte und im wesentlichen nicht turbulente Blutströmung vom Einlaßanschluß 34 durch die erfindungsgemäß vorgesehene Pumpenkammer 31 zum Auslaßanschluß 44 bei minimaler Beeinträchtigung der lebenden Blutbestandteile. Insoweit gewährleistet dieses Merkmal die Funktion der erfindungsgemäßen Blutpumpe bei einfachstem Aufbau.

Der auslaßseitige Kopf 43 ist mit einer Auslaß-Ventileinrichtung 47 versehen. Die Aufgabe dieser Auslaß-Ventileinrichtung 47 ist es, bei einem Überdruck innerhalb der Pumpenkammer 31 eine Blutströmung aus der Pumpenkammer 31 durch den Abgabeschlauch 50 zu ermöglichen, und bei einem Unterdruck innerhalb der Pumpenkammer 31 eine rückwärts gerichtete Strömung der Blutsäule aus dem Abgabeschlauch 50 in die Pumpenkammer 31 zu verhindern. Im Zusammenwirken von Einlaß-Ventileinrichtung 37 und Auslaß-Ventileinrichtung 47 darf immer nur eine Blutströmung in Richtung vom Einlaßanschluß 34 durch die Pumpenkammer 31 hindurch zum Auslaßanschluß 44 und in den Abgabeschlauch 50 auftreten. Folglich ist die Auslaß-Ventileinrichtung 47 im Übergangsbereich zwischen auslaßseitigem Kopf 43 und Auslaßanschluß 44 angeordnet und ausgebildet. Zu diesem Zweck kann der auslaßseitige Kopf 43 zweiteilig ausgebildet sein und aus zwei Hälften bestehen, die nach Anbringung und Ausbildung der Auslaß-Ventileinrichtung 47 innerhalb oder zwischen diesen beiden Hälften miteinander verklebt werden. Auch diese Auslaß-Ventileinrichtung 47 kann vergleichsweise einfach ausgebildet sein und kann vorzugsweise ein Rückschlagventil sein, das ein bewegliches Ventilglied aufweist, das im wesentlichen aus einer Klappe, einer Fahne, einem Segel oder einer Membran 47 besteht, die mit ihrem Umfangsrandabschnitt auf einer als Ventilsitzfläche dienenden Auflagefläche 48 aufliegen kann, die am auslaßseitigen Kopf 43 ausgebildet ist.

Diese Pumpeneinheit oder Pumpeinrichtung 30, bestehend aus dem Schlauchstück 32, dem einlaßseitigen Kopf 33 und dem auslaßseitigen Kopf 43 bildet ein unabhänig handhabbares Bauteil, das für den einmaligen Gebrauch bestimmt ist und das einfach und schnell in eine Blutpumpe einsetzbar ist, und das nach Gebrauch leicht gegen eine neue Pumpeinrichtung gleicher oder ähnlicher Bauart austauschbar ist. Zu diesem Zweck können der einlaßseitige Kopf 33 und der auslaßseitige Kopf 43 mit Befestigungsmittel versehen sein.

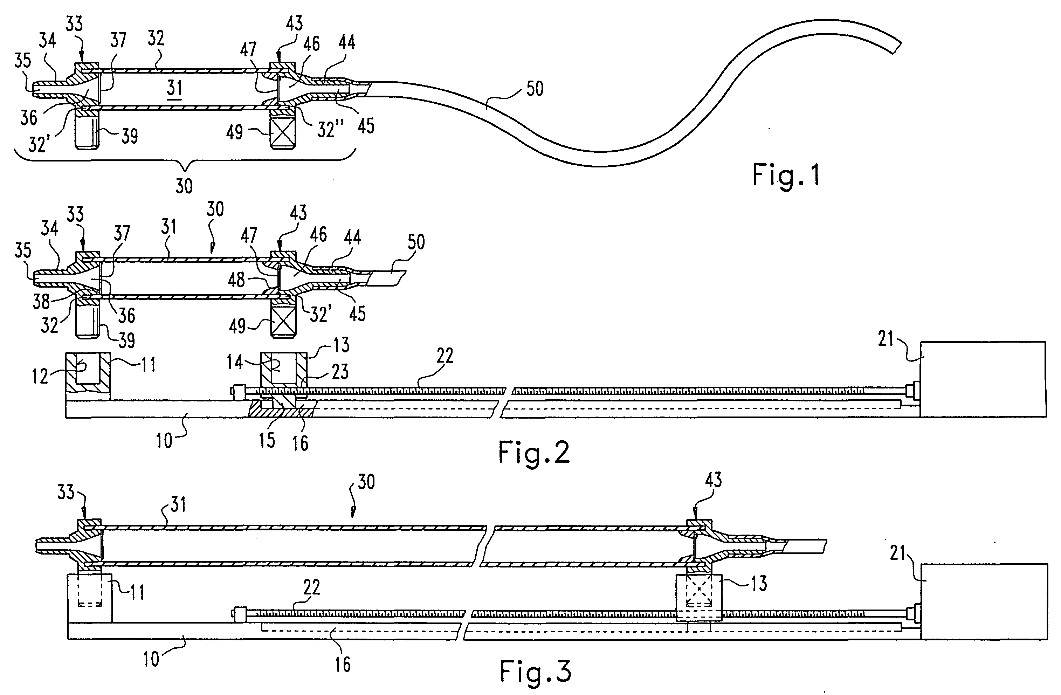

Beispielsweise kann der einlaßseitige Kopf mit einem einstückig angeformten Ansatz 39 versehen sein, der in eine Aufnahme 11 einsetzbar ist, die stationär an dem Gestell 10 angebracht ist. Weiterhin kann der auslaßseitige Kopf 43 mit einem einstückig angeformten Ansatz 49 versehen sein, der in eine Aufnahme 13 einsetzbar ist, die beweglich an dem Gestell angebracht ist, wie in Figur 2 dargestellt. Um die Unterscheidung zwischen den einlaßseitigen Kopf 33 und dem auslaßseitigen Kopf 43 zu erleichtern, kann der Ansatz 39 am einlaßseitigen Kopf 33 eine bestimmte, beispielsweise zylindrische Außenkontur aufweisen, und die stationäre Aufnahme 11 kann beispielsweise eine Aussparung 12 aufweisen, die mit einer angepaßten zylindrischen Innenkontur versehen ist. Der Ansatz 49 am auslaßseitigen Kopf 43 kann eine bestimmte andere, beispielsweise mehreckige Außenkontur aufweisen, und die verschieblich angeordnete Aufnahme 13 kann eine Aussparung 14 aufweisen, die eine angepaßte mehreckige Innenkontur aufweist.

Zu anderen Befestigungseinrichtungen gehört beispielsweise je eine Durchgangsbohrung 66, die am einlaßseitigen Kopf 33 ausgespart ist, und eine Durchgangsbohrung 64, die am auslaßseitigen Kopf 43 ausgespart ist. Wie das in Figur 4a dargestellt ist, kann der einlaßseitige Kopf 33 auf einen stationär angeordneten Stift 65 aufgesteckt werden, der sich durch diese Durchgangsbohrung 66 erstreckt, so daß eine stationäre, jedoch schwenkbare Anordnung des einlaßseitigen Kopfes 33 geschaffen wird. In gleicher Weise kann der auslaßseitige Kopf 43 auf ein umlaufendes Umfangsglied 33 aufgesteckt werden, das beispielsweise als Stift ausgebildet ist und sich durch die Durchgangsbohrung 64 hindurch erstreckt, so daß eine bewegliche Anordnung des auslaßseitigen Kopfes 43 geschaffen wird.

Zur Blutpumpe gehört weiterhin ein längliches Gestell 10, das beispielsweise in Form eines Stabes, einer Schiene, eines Profils, eines Rohres oder dergleichen ausgeführt sein kann. An einem Ende des Gestells ist eine Aufnahme 11 ortsfest angebracht, in welche ein Ansatz 39 eingeführt werden kann, der einstückig am einlaßseitigen Kopf 30 ausgebildet ist. Typischerweise weist diese Aufnahme 11 eine Aussparung, ein Loch oder eine Bohrung 12 auf, die mit einer bestimmten Innenkontur versehen ist, welche an die Außenkontur des Ansatzes 39 angepaßt ist. Weiterhin weist das Gestell 10 eine verschieblich angeordnete Aufnahme 13 auf, in welche ein Ansatz 49 eingesetzt werden kann, der einstückig mit dem auslaßseitigen Kopf 43 ausgebildet ist. Typischerweise kann diese beweglich angeordnete Aufnahme 13 eine Aussparung, ein Loch, eine Bohrung 14 aufweisen, die mit einer bestimmten Innenkontur versehen ist, die an die Außenkontur des Ansatzes 49 angepaßt ist. Bei einer beispielhaften Ausführungsform eines Antriebs ist diese verschieblich angeordnete Aufnahme 13 längs der Längsrichtung des länglichen Gestells 10 angeordnet. Hierzu kann am Gestell 10 eine in Längsrichtung verlaufende Nut 16 oder sonstige Führung ausgebildet sein, in welche verschieblich ein Fuß 15 eingreift, der einstückig mit der beweglichen Aufnahme 13 verbunden ist.

Weiterhin weist die erfindungsgemäße Blutpumpe einen Antrieb auf, der an dem Gestell 10 angebracht ist und der den auslaßseitigen Kopf verschieben oder verstellen kann. Nach einer beispielhaften, in den Figuren 2 und 3 dargestellten Ausführungsform weist dieser Antrieb einen elektrischen Schrittmotor 21 auf, der an einem gegenüberliegenden Ende des Gestells 10 angebracht ist, der motorisch eine Spindel 22 antreibt, die mit einem schraubenförmigen Außengewinde versehen ist. Diese Spindel 22 greift in eine Mutter oder einen sonstigen Gewindeabschnitt 23 ein, der mit dem verschieblich angeordneten Fuß 15 gekoppelt ist. Bei einer Drehung der Spindel 22 führt die verschieblich angeordnete Aufnahme 13 eine Verschiebung in Längsrichtung des Gestells 10 durch. Das Drehmoment und die Umdrehungsgeschwindigkeit des Schrittmotors 21, sowie die Steigung des Gewindes an der Spindel 22 werden vorzugsweise so ausgewählt und aufeinander abgestimmt, daß die bewegliche Aufnahme 13 einen vollständigen Hub, bestehend aus Hin- und Herbewegung in etwa 1 bis 3 sec ausführen kann. Typischerweise kann sich ein solcher vollständiger Hub über eine Distanz von etwa 100 bis 200 mm erstrecken. Es kann ein umsteuerbarer Schrittmotor 21 vorgesehen werden, um sowohl die Hinbewegung (in Ausziehrichtung des SchlauchstüKkes 32) wie die Herbewegung der beweglichen Aufnahme 13 unter der Einwirkung und Kontrolle der motorisch angetriebenen Spindel 22 durchzuführen. Nach einer alternativen Ausführungsform kann die motorisch angetriebene Spindel 22 lediglich die Hinbewegung der verschieblich angeordneten Aufnahme 13 durchführen, um das Schlauchstück 32 zu strekken. Eine entsprechende Her- oder Rückführbewegung der Aufnahme 13 wird durch eine elastische Rückstellkraft des Materials des gedehnten Schlauchstückes 32 verursacht. In diesem Falle ist der elektrische Schrittmotor 21 vorzugsweise und zusätzlich mit einer elektrischen Bremse ausgerüstet, welche auch bei dieser Rückführung der beweglichen Aufnahme 13 eine kontrollierte Umdrehungsgeschwindigkeit der Spindel 22 und damit eine kontrollierte Geschwindigkeit der Rückführung der beweglichen Aufnahme 13 gewährleistet.

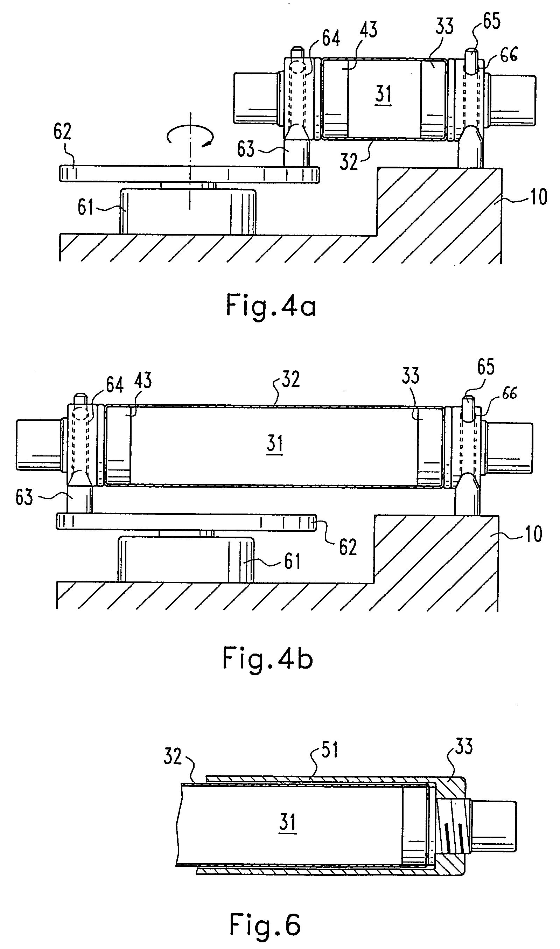

Mit den Figuren 4a und 4b ist eine weitere beispielhafte Ausführungsform eines Antriebs dargestellt. Dieser Antrieb ist am Gestell 10 befestigt und weist einen Elektromotor 61 auf, der eine rotierbare Scheibe 62 antreibt, die mit einem Umfangsglied 63 versehen ist. Dieses Umfangsglied 63 kann ein Stift oder Bolzen sein, der in einem Umfangsabschnitt der Scheibe 62 angebracht ist und dort vertikal von der Scheibe 62 absteht. Alternativ kann dieses Umfangsglied ein abstehendes Teil eines - nicht dargestellten - Kurbelbolzens sein, der von diesem Elektromotor 61 angetrieben wird. Folglich läuft das Umfangsglied 63 längs einer Kreisbahn um.

Eine Pumpeinheit weist je einen einlaßseitigen Kopf 33, eine Pumpenkammer 31 und und einen auslaßseitigen Kopf 43 auf. Dieser einlaßseitige Kopf 33 ist stationär an dem Gestell 10 angeordnet, und dieser auslaßseitige Kopf 43 ist mit diesem Umfangsglied 63 gekoppelt; beispielsweise kann der auslaßseitige Kopf 43 eine Durchgangsbohrung 64 aufweisen, und das Umfangsglied 63 ist durch diese Durchgangsbohrung 64 hindurch geführt. Der einlaßseitige Kopf 33 kann eine Durchgangsbohrung 66 aufweisen, und ist schwenkbar an einem weiteren Bolzen 65 angelenkt, der stationär an dem Gestell 10 angebracht ist. Ein Umlauf des Umfangsgliedes 63 verursacht eine aufeinanderfolgende Streckung und Verkürzung des Schlauchstückes 32, das die Pumpenkammer 31 begrenzt. Die Figur 4a zeigt dieses Schlauchstück 32 in einem im wesentlichen nicht gedehnten Zustand, und die Figur 4b zeigt dieses Schlauchstück 32 in einem gedehnten Zustand.

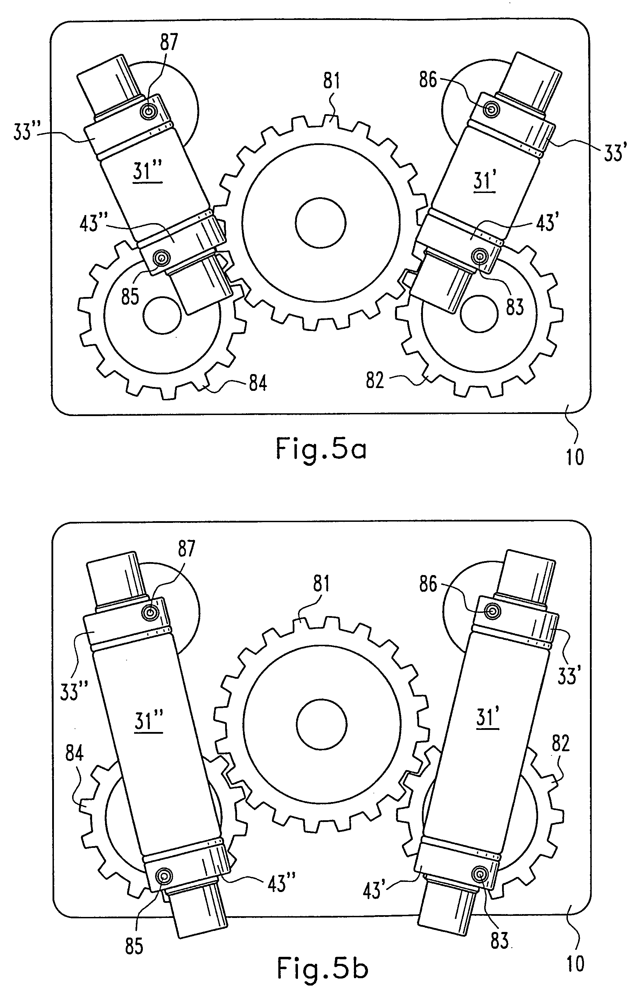

Mit den Figuren 5a und 5b ist noch eine weitere beispielhafte Ausführungsform eines Antriebs dargestellt. Dieser Antrieb weist ein motorisch angetriebenes Zahnrad 81 auf, das gleichzeitig ein erstes Zahnrad 82 und ein zweites Zahnrad 84 antreibt, die im Abstand zueinander angeordnet sind, die beide mit je einem Umfangsglied 83, 85 versehen sind, und die beide mit dem motorisch angetriebenen Zahnrad 81 kämmen. In jedem Falle kann dieses Umfangsglied 83, 85 ein Stift oder Bolzen sein, der vertikal von einem Umfangsabschnitt dieser Zahnräder 82, 84 absteht, und somit jeweils längs einer Kreisbahn umläuft, wenn das Zahnrad 81 die Zahnräder 82, 84 antreibt. Diese Ausführungsform einer erfindungsgemäßen Blutpumpe weist eine erste Pumpeinheit auf, mit einem ersten einlaßseitigen Kopf 33', einer ersten Pumpenkammer 31' und einem ersten auslaßseitigen Kopf 43'. Ferner weist diese Blutpumpe eine zweite Pumpeinheit auf, die mit einem zweiten einlaßseitigen Kopf 33", einer zweiten Pumpenkammer 31" und einem zweiten auslaßseitigen Kopf 43" versehen ist. Dieser erste auslaßseitige Kopf 43' ist mit dem Umfangsglied 83 am ersten angetriebenen Zahnrad 82 gekoppelt, und dieser zweite auslaßseitige Kopf 43" ist mit dem Umfangsglied 85 an dem zweiten angetriebenen Zahnrad 84 gekoppelt. Sowohl der erste einlaßseitige Kopf 33' wie der zweite einlaßseitige Kopf 33" sind unabhängig voneinander an je einem Bolzen 86 und 87 schwenkbar angelenkt, der an dem Gestell 10 stationär befestigt ist. Eine Drehung des Zahnrades 81 betätigt gleichzeitig die erste Pumpeinheit und die zweite Pumpeinheit. Figur 5a zeigt die erste und die zweite Pumpenkammer 31', 31" in einem im wesentlichen nicht gedehnten Zustand, und Fig. 5b zeigt diese erste und zweite Pumpenkammer 31', 31" in einem gedehnten Zustand.

In solchen Fällen, wo der Antrieb sowohl eine zwangsweise Streckung des Schlauchstückes 32 und eine zwangsweise Verkürzung des Schlauchstückes 32 bewirkt, verursacht dieser Verkürzungsschritt zumindestens einen leichten Überdruck innerhalb der Pumpenkammer 31, um zumindestens einen Teil des in der Pumpenkammer 31 befindlichen Blutes in die Abgabeleitung 50 zu drücken. Dieser Verkürzungs- oder Pumpschritt kann mit einer unerwünschten, übermäßigen Ausweitung oder Ausbeulung des Schlauchstückes 32 verbunden sein, insbesondere wenn das Schlauchstück 32 aus einem recht dünnen Material besteht, etwa einer dünnen Latexfolie. Wie das in Figur 6 dargestellt ist, kann ein starres Gehäuse oder Mantel 51 vorgesehen werden, das/der das nicht gedehnte Schlauchstück 32 umgibt. Dieser Mantel 51 kann einstückig mit dem einlaßseitigen Kopf 33 ausgebildet sein. Dieser Mantel 51 weist einen Innendurchmesser auf, der geringfügig größer ist, als der Außendurchmesser des nicht gedehnten Schlauchstückes 32, so daß ein übermäßiges Ausbeulen des Schlauchstückes 32 in dem vorstehend genannten Verkürzungs- oder Pumpschritt vermieden werden kann, selbst wenn dieses Schlauchstück aus einem sehr dünnen und flexiblen Material besteht.

Neben und zusätzlich zu den vorstehend genannten notwendigen Komponenten kann die erfindungsgemäße Blutpumpe mit weiteren wahlweise vorgesehenen Komponenten ausgerüstet sein. Solche wahlweise Komponenten können umfassen Sensoren zur Erfassung des Blutdruckes und des Blutdurchsatzes im Zuführungschlauch und/oder im Abgabeschlauch, Einrichtungen zur Einbringung von Heparin in das zugeführte Blut, eine Einrichtung zur Erfassung von Gasblasen im abgegebenen Blut und dergleichen. Derartige Einrichtungen sind für sich genommen in der Fachwelt bekannt und müssen hier nicht im einzelnen erläutert werden.

Mit der erfindungsgemäßen Blutpumpe kann frisches natürliches Blut auf besonders schonende Weise durch einen Dialyseapparat und/oder durch eine sonstige Einrichtung des extrakorporalen Blutkreislaufes gefördert werden.

## Patentansprüche

1. Extrakorporale Blutpumpe,
insbesondere zur Förderung von Blut durch einen Dialyseapparat,
mit einem Gestell (10), an dem eine Pumpeinrichtung (30) und ein Antrieb angebracht ist, wobei diese Pumpeinrichtung aufweist:
- einen an dem Gestell (10) der Blutpumpe stationär gehaltenen einlaßseitigen Kopf (33) mit Einlaßanschluß (34), Einlaßbohrung (35) und Einlaß-Ventileinrichtung (37),
- einen verschieblich angeordenten auslaßseitigen Kopf (43) mit Auslaßanschluß (44), Auslaßbohrung (45) und Auslaß-Ventileinrichtung (47),
- eine Pumpenkammer (31), deren Innenvolumen veränderbar ist, um eine Blutströmung vom Einlaßanschluß (34) durch die Pumpenkammer (31) zum Auslaßanschluß (44) zu erzeugen, und
- der Antrieb eine aufeinanderfolgende Vergrößerung und Verkleinerung des Innenvolumens der Pumpenkammer (31) bewirkt, wobei der Antrieb eine aufeinander folgende Hin- und Herbewegung des auslaßseitigen Kopfes (43) ausführt,
dadurch gekennzeichnet, daß
- die Pumpenkammer (31) aus einem Schlauchstück (32) aus Gummi-elastischem Material besteht, das ohne nennenswerte Einschnürung des Pumpenkammer-Innenvolumens auf ein Mehrfaches seiner ursprünglichen Länge ausziehbar ist, wobei ein Schlauchstückende (32') dicht und fest mit dem einlaßseitigen Kopf (33) sowie das gegenüberliegende andere Schlauchstückende (32") dicht und fest mit dem auslaßseitigen Kopf (43) verbunden ist; und
- pro Hub der Hin- und Herbewegung des auslaßseitigen Kopfes (43) im wesentlichen in Richtung der Schlauchstück-Längsachse, die ursprüngliche Länge des Schlauchstückes (32) um wenigstens 100 % und um nicht mehr als etwa 500 % der ursprünglichen Schlauchstücklänge gedehnt wird.

2. Blutpumpe nach Anspruch 1,
dadurch gekennzeichnet, daß
- die Einlaßbohrung (35) einen Innendurchmesser aufweist;
- die Auslaßbohrung (45) einen Innendurchmesser aufweist; und
- das die Pumpenkammer (31) begrenzende Schlauchstück (32) einen Innendurchmesser aufweist, der nicht größer ist als der fünffache Innendurchmesser der Einlaßbohrung (35) und/oder der Auslaßbohrung (45).

3. Blutpumpe nach Anspruch 2,
dadurch gekennzeichnet, daß
die ursprüngliche Länge des nicht gedehnten Schlauchstückes (32) größer ist als der Innendurchmesser des nicht gedehnten Schlauchstückes (32).

4. Blutpumpe nach einem der Ansprüche 1 bis 3,
dadurch gekennzeichnet, daß
der Antrieb eine aufeinanderfolgende Hin- und Herbewegung des auslaßseitigen Kopfes (43) im wesentlichen in Richtung der Schlauchstück-Längsrichtung ausführt, wobei pro Hub die ursprüngliche Länge des Schlauchstückes (32) um wenigstens 100 % und um nicht mehr als etwa 400 % der ursprünglichen Schlauchstücklänge gedehnt wird.

5. Blutpumpe nach einem der Ansprüche 1 bis 4,
dadurch gekennzeichnet, daß
- der auslaßseitige Kopf (43) aufeinanderfolgend eine Vorwärtsbewegung unter Streckung des Schlauchstückes (32) und eine Rückwärtsbewegung unter Verkürzung des Schlauchstückes (32) ausführt;
- die periodische Vorwärtsbewegung durch den Antrieb bewirkt wird; und
- die periodische Rückwärtsbewegung durch eine inhärente elastische Rückstellkraft des gedehnten Schlauchstückes (32) bewirkt wird.

6. Blutpumpe nach einem der Ansprüche 1 bis 5,
dadurch gekennzeihnet, daß
- eine periodische Vorwärtsbewegung des auslaßseitigen Kopfes (43) unter Streckung des Schlauchstückes (32) innerhalb der Pumpenkammer (31) eine Saugwirkung erzeugt;
- die Einlaß-Ventileinrichtung (37) ein Rückschlagventil ist, das unter dieser Saugwirkung eine Blutströmung aus einer an den Einlaßanschluß (34) angeschlossenen Versorgungsleitung durch die Einlaßbohrung (35) in die Pumpenkammer (31) erlaubt; und
- die Auslaß-Ventileinrichtung (47) ein Rückschlagventil ist, das unter dieser Saugwirkung die Auslaßbohrung (45) verschließt.

7. Blutpumpe nach einem der Ansprüche 1 bis 5,
dadurch gekennzeichnet, daß
- eine periodische Rückwärtsbewegung des auslaßseitigen Kopfes (43) unter Verkürzung des Schlauchstückes (32) innerhalb der Pumpenkammer (31) eine Pumpwirkung erzeugt;
- die Einlaß-Ventileinrichtung (37) ein Rückschlagventil ist, das unter dieser Pumpeinwirkung die Einlaßbohrung (35) verschließt; und
- die Auslaß-Ventileinrichtung (47) ein Rückschlagventil ist, das unter dieser Pumpwirkung eine Blutströmung aus der Pumpenkammer (31) durch die Auslaßbohrung (45) in eine Abgabeleitung (50) erlaubt, die an den Auslaßanschluß (44) angeschlossen ist.

8. Blutpumpe nach einem der Ansprüche 1 bis 7,
dadurch gekennzeichnet, daß
das Schlauchstück (31) aus einem Gummi-elastischen für medizinische Zwecke geeigneten Silikonmaterial oder Polyurethanmaterial oder Latexmaterial besteht.

9. Blutpumpe nach einem der Ansprüche 1 bis 8,
dadurch gekennzeichnet, daß
- die Blutpumpe ein längliches Gestell (10) aufweist, das an einem Ende eine stationäre Aufnahme (11) zur Halterung des einlaßseitigen Kopfes (33) aufweist; und
- dieses Gestell (10) ferner eine bewegliche Aufnahme (13) aufweist, die in einer sich in Gestell-Längsrichtung erstreckenden Nut oder Führung (16) geführt und zur Halterung des auslaßseitigen Kopfes (43) bestimmt ist.

10. Blutpumpe nach Anspruch 9,
dadurch gekennzeichnet, daß
- der einlaßseitige Kopf (33) mit einem einstückig angeformten Ansatz (39) versehen ist, der in die stationäre Aufnahme (11) einsetzbar ist; und
- der auslaßseitige Kopf (43) mit einem einstückig angeformten Ansatz (49) versehen ist, der in die bewegliche Aufnahme (13) einsetzbar ist.

11. Blutpumpe nach einem der Ansprüche 1 bis 10,
dadurch gekennzeichnet, daß
der Antrieb ein elektrischer Schrittmotor (21) ist, der eine Spindel (22) motorisch antreibt, die in eine Mutter (23) eingreift, welche mit dem auslaßseitigen Kopf (43) gekoppelt ist.

12. Blutpumpe nach Anspruch 12,
dadurch gekennzeichnet, daß
die Rückwärtsbewegung des auslaßseitigen Kopfes (43) unter Verkürzung des Schlauchstückes (32) durch die elastische Rückstellkraft des Materials des gedehnten Schlauchstückes (32) bewirkt wird; und der Schrittmotor (21) mit einer Strombremse versehen ist, welche bei dieser Rückführbewegung die Umdrehungsgeschwindigkeit der Spindel (22) kontrolliert.

13. Blutpumpe nach einem der Ansprüche 1 bis 9,
dadurch gekennzeichnet, daß
- der Antrieb einen Motor (61) aufweist, der eine rotierbare Scheibe (62) antreibt, von deren Umfangsbereich ein Umfangsglied (63) absteht; oder
- dieser Motor einen Kurbelbolzen antreibt, der ein abstehendes Umfangsglied aufweist;
- dieses Umfangsglied (63) längs einer Kreisbahn umläuft;
- der auslaßseitige Kopf (43) mit diesem umlaufenden Umfangsglied (63) gekoppelt ist; und
- ein Umlauf dieses Umfangsgliedes (63) je eine aufeinanderfolgende Streckung oder Verkürzung des Schlauchstückes (32) bewirkt.

14. Blutpumpe nach einem der Ansprüche 1 bis 9,
dadurch gekennzeichnet, daß
- der Antrieb ein motorisch angetriebenes Zahnrad (81) aufweist, das gleichzeitig ein erstes Zahnrad (82) und ein zweites Zahnrad (84 antreibt, die im Abstand zueinander angeordnet sind, mit dem motorisch angetriebenen Zahnrad kämmen und je ein abstehendes Umfangsglied (83, 85) aufweisen, das je längs eine Kreisbahn umläuft;
- diese Blutpumpe eine erste Pumpeinrichtung mit erstem einlaßseitigem Kopf (33'), erster Pumpenkammer (31') und erstem auslaßseitigem Kopf (43') aufweist, sowie eine zweite Pumpeinrichtung mit zweitem einlaßseitigem Kopf (33"), zweiter Pumpenkammer (31") und zweitem auslaßseitigem Kopf (43") aufweist;
- der erste auslaßseitige Kopf (43') mit dem umlaufenden Umfangsglied (83) am ersten Zahnrad (82) gekoppelt ist;
- der zweite auslaßseitige Kopf (43") mit dem umlaufenden Umfangsglied (85) am zweiten Zahnrad (84) gekoppelt ist;
so daß gleichzeitig eine erste Pumpeinrichtung und eine zweite Pumpeinrichtung betrieben werden.

15. Blutpumpe nach einem der Ansprüche 1 bis 14,
dadurch gekennzeichnet, daß
der einlaßseitige Kopf (33) mit einem starren abstehenden Gehäuse oder Mantel (51) versehen ist, das/der das nicht gedehnte Schlauchstück (32) umgibt und eine übermäßige Aufweitung dieses Schlauchstückes während einer Verkürzung oder eines Pumpschrittes der Pumpenkammer (31) verhindert.

## Claims

1. An extra-corporal blood pump,
especially for transportation of blood through a dialyzer apparatus, said blood pump comprising a support means (10) equipped with a pumping means (30) and a driving means,
wherein said pumping means comprises:
- an inlet head (33) stationarily arranged at said support means (10) and comprising an inlet connector (34), an inlet bore (35), and an inlet valve means (37),
- an outlet head (43) movably arranged and comprising an outlet connector (44), an outlet bore (45) and an outlet valve means (47),
- a pump chamber (31) having a variable interior volume in order to generate an enforced blood flow from the inlet connector (34) through the pump chamber (31) to the outlet connector (44), and
- said driving means effects a consecutive enlargement and reduction of the interior volume of said pump chamber (31), wherein said driving means performs a consecutive forward and backward movement of said outlet head (43),
**characterized in** that
- said pump chamber (31) is consisting of a piece (32) of a hose made of a rubber-elastic material which is extendable to a multiple length of it s original length without a substantial constriction of the interior volume of the pump chamber (31), wherein an end (32') of said hose piece is tightly connected with said inlet head (33) and wherein another distant end (32") of said hose piece is tightly connected with said outlet head (43); and
- per stroke of the forward and backward movement of said outlet head (43) essentially in a direction of a longitudinal axis of said hose piece (32), the original length of said hose piece (32) is extended by at least 100 % and by not more than about 500 %, each of the original hose piece length.

2. The extra-corporal blood pump according to claim 1,
**characterized in**, that
- said inlet bore (35) comprises an internal diameter;
- said outlet bore (45) comprises an internal diameter; and
- said hose piece (32) defining said pump chamber (31) comprises an internal diameter not larger than five-times the internal diameter of the inlet bore (35) and/or the outlet bore (45).

3. The extra-corporal blood pump according to claim 2,
**characterized in** that
the given original length of the non-extended hose piece (32) is larger than the internal diameter of said non-extended hose piece (32).

4. The extra-corporal blood pump according to anyone of the claims 1 to 3,
**characterized in** that
said driving means performs a consecutive forward and backward movement of said outlet head (43) essentially in a length direction of said hose piece thus extending per stroke the original length of the hose piece (32) by at least 100 % and by not more than about 400 %, each of the original hose piece length.

5. The extra-corporal blood pump according to anyone of the claims 1 to 4,
**characterized in** that
- said outlet head (43) is consecutively performing a forward movement causing a stretching of the hose piece (32), and a backward movement causing a shortening of the hose piece (32) ;
- said periodically moving forward being provided by said driving means; and
- said periodically moving backward being provided by an inherent elastic restoring force of the stretched hose piece (32).

6. The extra-corporal blood pump according to anyone of the claims 1 to 5,
**characterized in** that
- said periodically moving forward said outlet head (43) provides a stretching of said hose piece (32) and generates a suction action within the pump chamber (31) ;
- said inlet valve means (37) comprises a check valve allowing under said suction action a blood flow from a supply line connected to said inlet connector (34) through said inlet bore (35) into said pump chamber (31); and
- said outlet valve means (47) comprises a check valve closing under said suction action the outlet bore (45).

7. The extra-corporal blood pump according to anyone of the claims 1 to 5,
**characterized in** that
- said periodically moving backward said outlet head (43) provides a shortening of the hose piece (32) and generates a pumping action within the pump chamber (31);
- said inlet valve means (37) comprises a check valve closing under said pumping action the inlet bore (35); and
- said outlet valve means (47) comprises a check valve allowing under said pumping action a blood flow out of the pump chamber (31) through the outlet bore (45) into a dispensing line connected to the outlet connector (44).

8. The extra-corporal blood pump according to anyone of the claims 1 to 7,
**characterized in** that
said hose piece (32) is made of a rubber-elastic material suited for medical purposes and selected from a group comprising silicon materials, polyurethane materials and latex materials.

9. The extra-corporal blood pump according to anyone of the claims 1 to 8,
**characterized in** that
- said blood pump comprises an elongated support means (10) having a stationary retaining means (11) arranged adjacent to an end portion of said support means (10) and enabled to fasten said inlet head (33); and
- said support means (10) further comprises a movably arranged retaining means (13) guided along a groove or guidance (16) extending in a longitudinal direction of said support means (10) and enabled to fasten said outlet head (43).

10. The extra-corporal blood pump according to claim 9,
**characterized in** that
- said inlet head (33) comprises a one-piece formed fastening extension (39) insertable into said stationary retaining means (11); and
- said outlet head (43) comprises a one-piece formed fastening extension (49) insertable into said movably arranged retaining means (13).

11. The extra-corporal blood pump according to anyone of the claims 1 to 10,
**characterized in** that
said driving means comprises an electric step motor (21) driving a threaded spindle (22) which engages a nut (23) coupled with said outlet head (43).

12. The extra-corporal blood pump according to claim 11,
**characterized in** that
the backward movement of the outlet head (43) under shortening the hose piece (32) is caused by the inherent elastic restoring force of the material of the stretched hose piece (32); and said electric step motor (21) comprises an electric brake means controlling a rotational speed of the threaded spindle (22) during the backward movement of the outlet head (43).

13. The extra-corporal blood pump according to anyone of the claims 1 to 9,
**characterized in** that
- said driving means comprises a motor (61) driving a rotating disc (62) having a peripheral link (63) ; or
- said motor (61) driving a crank bolt having a peripheral link;
- said peripheral link (63) revolves along a circle path;
- said outlet head (43) being coupled with said peripheral link (63); and
- a revolution of said peripheral link (63) causes each a consecutive stretching and shortening of the hose piece (32).

14. The extra-corporal blood pump according to anyone of the claims 1 to 9,
**characterized in** that
- said driving means comprises a motor-driven toothed wheel (81) simultaneously driving a first gear wheel (82) and a second gear wheel (84) arranged distantly to each other and both comprising each a peripheral link (83, 85) ;
- the blood pump comprises a first pumping means having a first inlet head (33'), a first pump chamber (31') and a first outlet head (43'), and further comprises a second pumping means having a second inlet head (33"), a second pump chamber (31") and a second outlet head (43");
- said first outlet head (43') being coupled to the revolving peripheral link (83) of the first driven gear wheel (82);
- said second outlet head (43") being coupled to the revolving peripheral link (85) of the second driven gear wheel (84) ;
thus simultaneously operating the first pumping means and the second pumping means.

15. The extra-corporal blood pump according to anyone of the claims 1 to 14,
**characterized in** that
said inlet head (33) comprises a rigid envelope or casing (51) surrounding the non-stretched hose piece (32) and avoiding an undue bulging of said hose piece during a shortening step or pumping step of the pump chamber (31).

## Revendications

1. Pompe sanguine extracorporelle,
en particulier pour le transport du sang à travers un appareil de dialyse (10) avec un bâti sur lequel est installé un dispositif de pompe (30) et un entraînement, ce dispositif de pompe présentant
- une tête (33) maintenue côté entrée stationnaire sur le bâti (10) de la pompe sanguine avec un raccord d'admission (34) , un alésage d'admission (35) et un dispositif de soupape d'admission (37),
- une tête (43) de sortie disposée de manière mobile avec un raccordement de sortie (44), un alésage de sortie (45) et un dispositif de soupape de sortie (47),
- une enceinte de pompe (31) dont le volume intérieur est variable pour générer un écoulement de sang à travers l'enceinte de pompe (31) entre le raccord d'admission (34) et le raccordement de sortie (44) et
- l'entraînement provoque un agrandissement et une réduction successifs du volume intérieur de l'enceinte de pompe (31), l'entraînement exécutant un mouvement de va-et-vient consécutif de la tête (43) côté sortie,
caractérisée en ce que
- l'enceinte de pompe (31) se compose d'une pièce de tuyau flexible (32) en matière caoutchouc élastique qui est extensible sans étranglement notable du volume intérieur de l'enceinte de pompe à un multiple de sa longueur initiale, une extrémité de la pièce de tuyau flexible (32') étant reliée de manière étanche et fixe à la tête côté admission (33) ainsi que l'autre extrémité opposée de pièce de tuyau flexible (32") étant reliée de manière étanche et fixe à la tête côté sortie (43) ; et
- par course, le mouvement de va-et-vient de la tête côté admission (43) étendant sensiblement en direction de l'axe longitudinal de la pièce de flexible la longueur initiale de la pièce de tuyau flexible (32) d'au moins 100 % et de pas plus d'environ 500 % de la longueur initiale de la pièce de tuyau flexible.

2. Pompe sanguine selon la revendication 1,
caractérisée en ce que
- l'alésage d'admission (35) présente un diamètre interne ;
- l'alésage de sortie (45) présente un diamètre interne ; et
- la pièce de tuyau flexible limitant la chambre de pompe (31) présente un diamètre interne qui n'est pas supérieur au quintuple du diamètre interne de l'alésage d'admission (35) et/ou de l'alésage de sortie (45).

3. Pompe sanguine selon la revendication 2,
caractérisée en ce que
la longueur initiale de la pièce de tuyau flexible (32) non étirée est supérieure au diamètre interne de la pièce de tuyau flexible non étirée (32).

4. Pompe sanguine selon l'une des revendications 1 à 3,
caractérisée en ce que
l'entraînement exécute un mouvement consécutif de va-et-vient de la tête côté sortie (43) sensiblement en direction de la direction longitudinale de la pièce de tuyau flexible, par course la longueur initiale de la pièce de flexible (32) étant étendue d'au moins 100 % et de pas plus d'environ 400 % de la longueur initiale de la pièce de tuyau flexible.

5. Pompe sanguine selon l'une des revendications 1 à 4,
caractérisée en ce que
- la tête côté admission (43) exécute à la suite un mouvement vers l'avant sous l'extension de la pièce de flexible (32) et un mouvement vers l'arrière en raccourcissant de la pièce de tuyau flexible (32) ;
- le mouvement vers l'avant périodique est provoqué par l'entraînement ; et
- le mouvement vers l'arrière périodique est provoqué par une force de rappel élastique inhérente de la pièce flexible étirée (32).

6. Pompe sanguine selon l'une des revendications 1 à 5
caractérisée en ce qu'
- un mouvement périodique d'avance de la tête côté sortie (43) génère par extension de la pièce de tuyau flexible (32) un effet d'aspiration à l'intérieur de l'enceinte de pompe (31) ;
- le dispositif de soupape d'entrée (37) est une soupape de non retour qui permet sous cet effet d'aspiration un écoulement de sang sortant de la conduite d'alimentation raccordée au raccordement d'admission (34) par l'alésage d'admission (35) dans l'enceinte de pompe (31) ; et
- le dispositif de soupape de sortie (47) est une soupape de non retour qui ferme l'alésage de sortie (45) sous cet effet d'aspiration.

7. Pompe sanguine selon l'une des revendications 1 à 5,
caractérisée en ce qu'
- un mouvement périodique vers l'arrière de la tête côté sortie (43) génère un effet de pompage à l'intérieur de l'enceinte de pompe (31) en réduisant la pièce de tuyau flexible (32) ;
- le dispositif de soupape d'admission (37) est une soupape de non retour qui ferme sous cet effet de pompage l'alésage d'admission (35) ; et
- le dispositif de soupape de sortie (47) est une soupape de non retour qui permet sous cet effet de pompage un écoulement de sang sortant de l'enceinte de pompe (31) par l'alésage de sortie (45) dans une conduite de distribution (50) qui est raccordée au raccordement de sortie (44).

8. Pompe sanguine selon l'une des revendications 1 à 7,
caractérisée en ce que
la pièce de tuyau flexible (31) se compose de matériau à base de silicone ou de polyuréthane en caoutchouc élastique convenant à un usage médical ou bien en latex.

9. Pompe sanguine selon l'une des revendications 1 à 8,
caractérisée en ce que
- la pompe sanguine présente un bâti allongé (10) qui présente à une extrémité un logement stationnaire (11) pour le maintien de la tête côté entrée (33) ; et
- ce bâti (10) présente en outre un logement mobile (13) qui est guidé dans une rainure ou un guide (16) s'étendant dans le sens longitudinal du bâti et qui est destiné à fixer la tête côté sortie (43).

10. Pompe sanguine selon la revendication 9,
caractérisée en ce que
- la tête côté admission (33) est munie d'un épaulement (39) moulé d'une seule pièce qui peut être inséré dans le logement stationnaire (11) ; et
- la tête côté sortie (43) est munie d'un épaulement (49) moulé d'une seule pièce qui peut être inséré dans le logement mobile (13).

11. Pompe sanguine selon l'une des revendications 1 à 10,
caractérisée en ce que
l'entraînement est un moteur électrique (21) pas à pas qui entraîne une broche (22) motorisée qui s'engage dans un écrou (23) accouplé à la tête côté sortie (43).

12. Pompe sanguine selon la revendication 11,
caractérisée en ce que
le mouvement vers l'arrière de la tête côté sortie (43) est provoqué par réduction de la pièce de tuyau flexible (32) par la position élastique de rappel du matériau de la pièce de tuyau flexible étirée (32) ; et
le moteur pas à pas (21) est muni d'un frein rhéostatique qui contrôle lors de ce mouvement vers l'arrière la vitesse de rotation de la broche (22).

13. Pompe sanguine selon l'une des revendications 1 à 8,
caractérisée en ce que
- l'entraînement présente un moteur (61) qui entraîne un disque rotatif (62) dont un organe périphérique (63) fait saillie de la zone périphérique du disque ; ou
- ce moteur entraîne un boulon à manivelle qui présente un organe périphérique saillant ;
- cet organe périphérique (63) fait le tour d'une orbite ;
- la tête côté sortie (43) est accouplée à cet organe périphérique tournant ; et
une rotation de cet organe circulaire (63) provoque une extension ou une réduction consécutive de la pièce de tuyau flexible (32).

14. Pompe sanguine selon l'une des revendications 1 à 8,
caractérisée en ce que
- l'entraînement présente une roue dentée motorisée (81) qui entraîne en même temps une première roue dentée (82) et une seconde roue dentée (84) qui sont disposées à distance l'une de l'autre, s'engrènent avec la roue dentée motorisée et présentent chacune un organe périphérique (83, 85) faisant saillie qui tourne le long d'une orbite ;
- cette pompe sanguine présente un premier dispositif de pompage avec une première tête côté entrée (33'), une première enceinte de pompe (31') et une première tête côté sortie (43') ainsi qu'un second dispositif de pompage avec une seconde tête côté sortie (33"), une seconde enceinte de pompage (31") et une seconde tête côté sortie (43") ;
- la première tête côté sortie (43') est accouplée à un organe périphérique tournant (83) sur la première roue dentée (82) ;
- la seconde tête côté sortie (43") est accouplée à l'organe périphérique tournant (85) sur la seconde roue dentée (84) ;
de sorte qu'un premier dispositif de pompage et un second dispositif de pompage sont entraînés en même temps.

15. Pompe sanguine selon l'une des revendications 1 à 14,
caractérisée en ce que
la tête côté admission (33) est munie d'un carter ou enveloppe rigide saillant (51) qui entoure la pièce de tuyau flexible non étendue et empêche un élargissement exagéré de cette pièce flexible pendant une réduction ou une étape de pompage de l'enceinte de pompage (31).
